# EUROPÄISCHE PATENTSCHRIFT

(11) **EP 1 330 424 B1**
(45) Veröffentlichungstag und Bekanntmachung des Hinweises auf die Patenterteilung: **15.09.2004**
(21) Anmeldenummer: 01976290.5
(22) Anmeldetag: 09.10.2001
(51) Int. Cl.: C07C 5/10, C07C 209/72

(54) **VERFAHREN ZUR HYDRIERUNG VON AROMATEN MITTELS REAKTIVDESTILLATION**
METHOD FOR THE HYDROGENATION OF AROMATICS BY MEANS OF REACTIVE DISTILLATION
PROCEDE D'HYDROGENATION DE COMPOSES AROMATIQUES PAR DISTILLATION REACTIVE

(30) Priorität: 13.10.2000 DE 10050711
(43) Veröffentlichungstag der Anmeldung: 30.07.2003
(73) Patentinhaber: BASF AKTIENGESELLSCHAFT, 67056 Ludwigshafen (DE)
(72) Erfinder: BÖTTCHER, Arnd, 67227 Frankenthal (DE); OOST, Carsten, 67098 Bad Dürkheim (DE); HAAKE, Mathias, 68161 Mannheim (DE)
(74) Vertreter: Isenbruck, Günter, Dr.
(86) Internationale Anmeldenummer: PCT/EP2001/011650
(87) Internationale Veröffentlichungsnummer: WO 2002/030855

(56) Entgegenhaltungen:
- WO-A-96/27580
- US-A- 5 599 997

## Beschreibung

Die vorliegende Erfindung betrifft ein Verfahren zur Hydrierung von gegebenenfalls mit mindestens einer Alkylgruppe, Aminogruppe oder Hydroxylgruppe oder einer Kombination aus zwei oder mehr davon substituierten ein- oder mehrkernigen Aromaten zu den entsprechenden Cycloaliphaten. Insbesondere betrifft die vorliegende Erfindung ein Verfahren zur Hydrierung von Benzol zu Cyclohexan mittels Reaktivdestillation in einer Reaktionskolonne unter Führung der Reaktionspartner im Gegenstrom über den/die in der Reaktionskolonne fixierten Katalysator(en).

Es existieren zahlreiche Verfahren zur Hydrierung von beispielsweise Benzol zu Cyclohexan. Diese Hydrierverfahren werden überwiegend an partikelförmigen Nickel- und Platinkatalysatoren in der Gas- oder Flüssigphase durchgeführt (s.u.a. US 3 597 489 bzw. GB 1 444 499 bzw. GB 992 104). Typischerweise wird dabei zunächst in einem Hauptreaktor der größte Teil des Benzols zu Cyclohexan hydriert und anschließend in einem oder mehreren Nachreaktoren die Umsetzung zu Cyclohexan komplettiert.

Die stark exotherme Hydrierreaktion erfordert eine sorgfältige Druck-, Temperatur- und Verweilzeitkontrolle, um einen vollständigen Umsatz bei einer hohen Selektivität zu erreichen. Insbesondere muß eine signifikante Bildung von Methylcyclopentan unterdrückt werden, die bevorzugt bei höheren Temperaturen abläuft. Typische Cyclohexan-Spezifikationen fordern einen Benzol-Restgehalt < 100 ppm und einen Methylcyclopentan-Gehalt < 200 ppm. Auch der Gehalt an n-Paraffinen (n-Hexan, n-Pentan u.a.) ist kritisch. Diese unerwünschten Verbindungen entstehen ebenfalls bevorzugt bei höheren Hydriertemperaturen und lassen sich ebenso wie Methylcyclopentan nur durch aufwendige Trennoperationen vom produzierten Cyclohexan abtrennen. Die Trennung kann beispielsweise durch Extraktion, Rektifikation oder durch Verwendung von Molekularsieben, wie in der GB 1 341 057 beschrieben, erfolgen. Auch der zur Hydrierung eingesetzte Katalysator hat einen starken Einfluß auf das Ausmaß der Bildung von unerwünschtem Methylcyclopentan.

Vor diesem Hintergrund ist es erstrebenswert, die Hydrierung möglichst bei niedrigen Temperaturen durchzuführen. Dies ist jedoch dadurch limitiert, daß in Abhängigkeit von der Art des verwendeten Hydrierkatalysators erst ab höheren Temperaturen eine zum Erreichen wirtschaftlicher Raum-Zeit-Ausbeuten genügend hohe Hydrieraktivität des Katalysators erreicht wird.

Die für die Benzolhydrierung eingesetzten Nickel- und Platinkatalysatoren weisen eine Reihe von Nachteilen auf. Nickelkatalysatoren sind sehr empfindlich gegenüber schwefelhaltigen Verunreinigungen im Benzol, so daß man entweder sehr reines Benzol zur Hydrierung einsetzen muß oder, wie in der GB 1 104 275 beschrieben, im Hauptreaktor einen Platinkatalysator einsetzt, der einen höheren Schwefelgehalt toleriert und so den Nachreaktor, der mit einem Nickelkatalysator gefüllt ist, schützt. Eine andere Möglichkeit besteht in der Dotierung des Katalysators mit Rhenium (GB 1 155 539) oder in der Herstellung des Katalysators unter Verwendung von Ionenaustauschern (GB 1 144 499). Die Herstellung derartiger Katalysatoren ist jedoch aufwendig und teuer. Auch an Raney-Nickel kann die Hydrierung durchgeführt werden (US 3 202 723); von Nachteil ist jedoch die leichte Brennbarkeit dieses Katalysators. Auch homogene Nickelkatalysatoren können zur Hydrierung eingesetzt werden (EP-A 0 668 257). Diese Katalysatoren sind jedoch sehr wasserempfindlich, so daß das eingesetzte Benzol vor der Hydrierung zunächst in einer Trocknungskolonne auf einen Restwassergehalt < 1 ppm getrocknet werden muß. Ein weiterer Nachteil des Homogen-Katalysators ist, daß dieser nicht mit vertretbarem Aufwand regeneriert werden kann.

Platinkatalysatoren weisen weniger Nachteile als Nickelkatalysatoren auf, sind jedoch viel teurer in ihrer Herstellung. Sowohl bei der Verwendung von Platin- als auch von Nickelkatalysatoren sind sehr hohe Hydriertemperaturen notwendig, was zu einer signifikanten Bildung unerwünschter Nebenprodukte führen kann.

An Ruthenium-Katalysatoren wird die Hydrierung von Benzol zu Cyclohexan technisch nicht ausgeübt; in der Patentliteratur finden sich jedoch Hinweise auf die Verwendung von rutheniumhaltigen Katalysatoren für diese Anwendung:

In der Druckschrift SU 319 582 werden Ru-Suspensionskatalysatoren, die mit Pd, Pt oder Rh dotiert sind, zur Herstellung von Cyclohexan aus Benzol eingesetzt. Die Katalysatoren sind jedoch durch die Verwendung von Pd, Pt oder Rh sehr teuer. Ferner ist bei Suspensionskatalysatoren die Aufarbeitung und Wiedergewinnung des Katalysators aufwendig und teuer.

In der Druckschrift SU 403 658 wird ein mit Cr dotierter Ruthenium-Katalysator zur Herstellung von Cyclohexan eingesetzt. Die Hydrierung erfolgt bei 180 C; hierbei wird eine signifikante Menge unerwünschter Nebenprodukte generiert.

In der Druckschrift US 3 917 540 werden auf Al₂O₃ als Trägermaterial aufgebrachte Katalysatoren zur Herstellung von Cyclohexan beansprucht. Diese enthalten als Aktivmetall ein Edelmetall aus der VIII. Nebengruppe des Periodensystems, weiterhin ein Alkalimetall sowie Technetium oder Rhenium. Die Al₂O₃-Träger liegen in Form von Kugeln, Granulaten oder ähnlichem vor. Nachteil derartiger Katalysatoren ist, daß lediglich eine Selektivität von 99,5 % erreicht wird.

In der Druckschrift US 3 244 644 werden schließlich auf η-Al₂O₃ als Trägermaterial aufgebrachte Ruthenium-Hydrierkatalysatoren beschrieben, die sich auch zur Hydrierung von Benzol eignen sollen. Die Katalysatoren sind in Partikeln von maximal 0,635cm (¼ inch) geformt und weisen mindestens 5 % Aktivmetall auf; die Herstellung von η-Al₂O₃ ist aufwendig und teuer.

Im Stand der Technik sind neben den vorstehend beschriebenen partikelförmigen Katalysatoren oder Suspensions-Katalysatoren monolithische Trägerkatalysatoren in Form von geordneten Packungen mit katalytisch aktiven Schichten bekannt, die für Hydrierreaktionen eingesetzt werden können.

In der Druckschrift EP-B 0 564 830 wird beispielsweise ein monolithischer Trägerkatalysator beschrieben, der als Aktivkomponenten Elemente der VIII. Gruppe des Periodensystems aufweisen kann.

Die Druckschrift EP-A 0 803 488 offenbart ein Verfahren zur Umsetzung, beispielsweise Hydrierung, einer aromatischen Verbindung, die mindestens eine Hydroxylgruppe oder Aminogruppe an einem aromatischen Ring trägt. Die Umsetzung wird durchgeführt in Gegenwart eines Katalysators, der eine homogene Rutheniumverbindung umfaßt, die sich in situ auf einem Träger, beispielsweise einem Monolithen, abgeschieden hat. Die Hydrierung wird bei Drücken von mehr als 50 bar und Temperaturen von vorzugsweise 150 °C bis 220 °C durchgeführt.

Die WO 96/27580 beschreibt ein Verfahren zur Hydrierung von ungesättigten cyclischen und polycyclischen Verbindungen mittels katalytischer Destillation, in dem der Reaktor bei einem Druck betrieben wird, bei dem die Reaktionsmischung unter niedrigem Wasserstoff-Partialdruck siedet.

Die WO 98/09930 offenbart ein Verfahren zur selektiven Hydrierung aromatischer Verbindungen in einem gemischten Kohlenwasserstoff-Strom mittels katalytischer Destillation in Gegenwart eines Katalysators.

Bei den Verfahren der beiden letztgenannten Druckschriften sind Drücke von 13,8 bis 17,2 bar und Temperaturen von 135 bis 190°C erforderlich, um eine ausreichende Raum-Zeit-Ausbeute zu erzielen. Gemäß beiden Druckschriften wird das gewünschte Produkt stets über Kopf abgezogen bzw. erhalten.

Alle in der Literatur beschriebenen Verfahren zur Hydrierung von aromatischen Verbindungen haben gemeinsam, daß die stark exotherme Hydrierreaktion eine sorgfältige Temperatur- und Verweilzeitkontrolle erfordert, um einen vollständigen Umsatz bei hoher Selektivität zu erreichen. Insbesondere muß eine signifikante Bildung von Methylcyclopentan, das bevorzugt bei hohen Temperaturen gebildet wird, unterdrückt werden. Die bei der Hydrierung entstehenden Nebenprodukte wie beispielsweise Methylcyclopentan führen bei den o.g. Verfahren des Standes der Technik zu einer Verunreingiung des Produkts. Zur Herstellung von beispielsweise hochreinem Cyclohexan ist daher gegebenenfalls eine nachfolgende Destillation erforderlich, die mit Investitionskosten verbunden ist.

Der vorliegenden Erfindung lag deshalb die Aufgabe zugrunde, ein wirtschaftliches Verfahren zur Herstellung von Cycloaliphaten durch Hydrierung der entsprechenden Aromaten, insbesondere durch Hydrierung von Benzol unter Erhalt von Cyclohexan, bereitzustellen, das es ermöglicht, Cycloaliphaten von hohem Reinheitsgrad mit hoher Selektivität, hoher Ausbeute und unter milden Reaktionsbedingungen zu erhalten.

Diese Aufgabe wird gelöst durch das erfindungsgemäße Verfahren zur Hydrierung von mit mindestens einer Alkylgruppe, Aminogruppe oder Hydroxylgrupe oder einer Kombination aus zwei oder mehr davon substituierten oder unsubstituierten ein- oder mehrkernigen Aromaten zu den entsprechenden Cycloaliphaten mit gasförmigem Wasserstoff in Gegenwart mindestens eines Katalysators in einer Reaktionskolonne unter Führung der Reaktionspartner über den(die) in der Reaktionskolonne fixierten Katalysator(en), wobei die Hydrierung derart durchgeführt wird, daß die Cycloaliphaten aus einem Seitenabzug oder dem Sumpf der Kolonne oder dem Seitenabzug und dem Sumpf der Kolonne entnommen werden.

Vorzugsweise werden die Reaktionspartner im Gegenstrom über den(die) in der Reaktionskolonne fixierten Katalysatoren) geführt.

Sofern die als Produkt erwünschten Cycloaliphaten über einen Seitenabzug entnommen werden, werden die leichter siedenden Komponenten (Leichtsieder) am Kopf der Kolonne abgezogen. Entsprechend werden die höher als der Cycloaliphat siedenden Komponenten (Hochsieder) am Sumpf der Kolonne erhalten. Demgemäß wird die Fahrweise an die jeweiligen Nebenprodukte, die in Aromaten vorhanden sind oder während der Reaktion entstehen, angepaßt. Beispielsweise werden Leichtsieder über Kopf abgezogen und die entsprechend hochsiedenden Komponenten über Sumpf, während der Cycloaliphat über einen Seitenabzug erhalten wird.

Falls keine hochsiedenden Nebenprodukte oder Nebenkomponenten anfallen, wird das Wertprodukt über Sumpf abgezogen.

Selbstverständlich ist erfindungsgemäß auch eine Fahrweise möglich, in der die Cycloaliphaten als Wertprodukte über den Seitenabzug und im Sumpf der Kolonne anfallen.

Dabei wird erfindungsgemäß über das Rücklaufverhältnis in der Kolonne und/oder dem Energieeintrag in die Kolonne gesteuert, ob die Cycloaliphaten am Seitenabzug oder im Sumpf der Kolonne anfallen. Am Seitenabzug wird das Produkt bevorzugt in flüssiger Form entnommen.

Dabei wurde überraschenderweise gefunden, daß sich Aromaten, für die als nicht beschränkendes Beispiel Benzol, Toluol, Xylole und Anilin genannt werden können, durch das erfindungsgemäße Verfahren bei, verglichen mit den Verfahren des Standes der Technik, deutlich niedrigeren Drücken und Temperaturen selektiv und mit hoher Raum-Zeit-Ausbeute zu den entsprechenden Cycloaliphaten hydrieren lassen und diese in hoher Reinheit in einer Vorrichtung gewonnen werden.

Im Rahmen des erfindungsgemäßen Verfahrens wird die Hydrierung vorzugsweise bei einem Druck < 20 bar und bei einer Temperatur < 200 °C durchgeführt.

In einer besonders bevorzugten Ausführungsform wird die Hydrierung bei einem Druck < 13 bar und bei einer Temperatur < 150°C durchgeführt.

Noch mehr bevorzugt wird die Hydrierung bei einem Druck im Bereich von 1 bis 20 bar, vorzugsweise 5 bis 13 bar und/oder bei einer Temperatur im Bereich von 50 bis 200 °C, vorzugsweise 80 bis 150 °C durchgeführt.

Da sich das System im Siedezustand befindet, kann bei dem erfindungsgemäßen Verfahren die Temperatur des Reaktionsgemisches auf einfache Weise über den Druck geregelt werden.

In dem erfindungsgemäßen Verfahren wird der Druck so eingestellt, daß bei der Hydrierung der Wasserstoff-Partialdruck im Bereich von 0,1 bis 20 bar und vorzugsweise im Bereich von 5 bis 13 bar liegt.

Bei dem erfindungsgemäßen Verfahren wird die katalytische Hydrierung in einer Reaktionskolonne an einem heterogenen Katalysator durchgeführt, wobei prinzipiell alle für diese Anwendung geeigneten Katalysatoren eingesetzt werden können.

Im einzelnen sind dabei beispielhaft zu nennen: Formkörper aus katalytisch wirksamen Ionentauschern, wie sie in Chem. Eng. Technol. 16 (1993), S. 279 bis 289 beschrieben sind, die in Form von Raschigringen, Sattelkörpern und anderen aus der Destillationstechnik bekannten Formen ausgeführt sind. Ein weiteres Beispiel für katalytisch aktive Formkörper, die in ihrer Gestaltung Trenneinbauten aus der Destillationstechnik ähneln, sind die von der Firma Sulzer hergestellten KATAPAK Katalysatoren und Katalysatorträger sowie die von der Firma Montz hergestellten MULTIPAK Katalysatoren. Sie stimmen in ihrer Geometrie mit den in der Destillationstechnik verbreiteten Kreuzkanalstrukturen, wie beispielsweise den Bauarten Sulzer BX, CY, DX, MELAPAK oder Montz A3, BSH überein. Ähnliche Geometrien, jedoch in Form von Drahtgeweben, die zusätzlich aufgerauht sind, werden in der DE-A 19624130.8 offenbart.

Ferner können Katalysatoren, wie z.B. Ionentauscher, die in Taschen von Drahtnetzen eingebettet sind, zu Ballen (engl. *Bales*) mit einem Durchmesser von etwa 0,2 bis 0,6 m aufgerollt werden, wobei die Höhe eines solchen Ballens etwa 0,3 m beträgt. Diese Ballen werden einzeln oder zu mehreren in die Destillationskolonnen eingebracht. Weitere Informationen bezüglich derartiger Katalysatoren sind in US 4 215 011 sowie in Ind. Eng.

Chem. Res. (1997), 36, S. 3821 bis 3832 zu entnehmen, deren diesbezüglicher Inhalt voll umfänglich in den Kontext der vorliegenden Anmeldung aufgenommen wird.

Ferner können Heterogenkatalysatoren mit Aktivmetallen eingesetzt werden. Als Aktivmetalle können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird.

Unter den ebenfalls verwendbaren Metallen der I. und der VII. Nebengruppe des Periodensystems, die ebenfalls allesamt prinzipiell verwendbar sind, werden vorzugsweise Kupfer und/oder Rhenium eingesetzt.

Besonders bevorzugt wird Ruthenium allein eingesetzt. Ein Vorteil der Verwendung des Hydriermetalls Ruthenium ist, daß hierdurch im Vergleich zu den deutlich teureren Hydriermetallen Platin, Palladium oder Rhodium erhebliche Kosten bei der Katalysatorherstellung eingespart werden können.

Der in dem erfindungsgemäßen Verfahren bevorzugt eingesetzte Ruthenium-Katalysator ist entweder in Form einer Schüttung oder als katalytisch aktive Destillationspackung oder in Kombination von beiden in der Kolonne plaziert. Die Form einer derartigen Schüttung oder Destillationspackung ist dem Fachmann aus dem Stand der Technik bereits bekannt.

Beispiele für metallische Werkstoffe als Trägermaterialien sind Reinmetalle wie Eisen, Kupfer, Nickel, Silber, Aluminium Zirkonium, Tantal und Titan oder Legierungen wie Stähle oder Edelstähle, wie z.B. Nickel-, Chrom- und/oder Molybdän-Stahl. Ferner können Messing, Phosphorbronze, Monell und/oder Neusilber oder Kombinationen aus zwei oder mehr der oben genannten Materialien eingesetzt werden.

Beispiele für keramische Werkstoffe sind Aluminiumoxid (Al₂O₃), Siliciumdioxid (SiO₂), Zirkoniumdioxid (ZrO₂), Cordierit und/oder Steatit.

Beispiele für synthetische Trägermaterialien sind beispielsweise Kunststoffe wie Polyamide, Polyester, Polyether, Polyvinyle, Polyolefine wie Polyethylen, Polypropylen, Polytetrafluorethylen, Polyketone, Polyetherketone, Polyethersulfone, Epoxidharze, Aldehydharze, Harnstoff- und/oder Melamin-Aldehydharze. Weiterhin kann Kohlenstoff als Träger eingesetzt werden.

Bevorzugterweise werden strukturierte Träger in Form von Metallgeweben, gestricken, -gewirken, -blechen oder -filzen, Kohlefasergeweben oder -filzen oder Kunststoffgeweben oder -gestricken verwendet. Als Drahtgewebe kommen Gewebe aus webbaren Metalldrähten wie Eisen, Federstahl, Messing, Phosphorbronze, Reinnickel, Monel, Aluminium, Silber, Neusilber, Nickel, Chromnickel, Chromstahl, nicht rostenden, säurebeständigen und hochhitzebeständigen Chromnickelstählen sowie Titan in Betracht.

Ebenfalls können Gewebe aus anorganischen Materialien verwendet werden, wie beispielsweise Gewebe aus keramischen Werkstoffen wie Al₂O₃ und/oder SiO₂.

Auch synthetische Drähte und Gewebe aus Kunststoffen sind gemäß einer Ausführungsform der Erfindung einsetzbar.

Monolithe aus Gewebepackungen sind besonders bevorzugt, da sie hohe Querschnittsbelastungen von Gas und Flüssigkeit aushalten und dabei einen nur unwesentlichen Abrieb zeigen.

In einer weiteren besonders bevorzugten Ausführungsform werden metallische, strukturierte Träger oder Monolithen verwendet, die aus Edelstahl bestehen, der vorzugsweise beim Tempern unter Luft und anschließendem Abkühlen eine Aufrauhung der Oberfläche zeigt. Diese Eigenschaften zeigen insbesondere Edelstähle, bei denen sich oberhalb einer spezifischen Entmischungstemperatur ein Legierungsbestandteil an der Oberfläche anreichert und in Gegenwart von Sauerstoff durch Oxidation eine festhaftende rauhe oxidische Oberflächenschicht bildet. Ein solcher Legierungsbestandteil kann beispielsweise Aluminium oder Chrom sein, aus denen sich entsprechend eine Oberflächenschicht aus Al₂O₃ oder Cr₂O₃ bildet. Beispiele für Edelstähle sind die mit den Werkstoff-Nummern 1.4767, 1.4401, 1.4301, 2.4610, 1.4765, 1.4847 und 1.4571. Diese Stähle können vorzugsweise durch Tempern an der Luft bei 400 bis 1.100 °C über einen Zeitraum von 1 Stunde bis zu 20 Stunden und anschließendes Abkühlenlassen auf Raumtemperatur thermisch aufgerauht werden.

In einer bevorzugten Ausführungsform ist der heterogene Katalysator ein mit Ruthenium beschichtetes Gewebe, das gleichzeitig als Destillationspackung wirkt.

In einer noch weiter bevorzugten Ausführungsform des erfindungsgemäßen Verfahrens besteht die Destillationspackung aus mit Ruthenium beschichteten Metallfäden, wobei besonders bevorzugt die Edelstähle der Nummer 1.4301 oder 1.4767 eingesetzt werden.

Wie aus dem Stand der Technik dem Fachmann bekannt, kann auch ein Promotor oder können auch mehrere Promotoren für den Katalysator verwendet werden. Die Promotoren können beispielsweise Alkali- und/oder Erdalkalimetalle, wie Lithium, Natrium, Kalium, Rubidium, Cäsium, Magnesium, Calcium, Strontium und Barium, Münzmetalle wie Kupfer, Silber und/oder Gold, Zink, Zinn, Bismuth, Antimon, Molybdän, Wolfram und/oder andere Promotoren wie Schwefel und/oder Selen sein.

Vor Aufbringung der Aktivmetalle und gegebenenfalls Promotoren können die strukturierten oder monolithischen Träger gegebenenfalls mit einem, zwei oder mehreren Oxiden beschichtet werden. Dies kann physikalisch, beispielsweise durch Sputtern, erfolgen. Hierbei werden in oxidierender Atmosphäre unter Hochvakuum-Bedingungen Elemente und/oder Elementverbindungen auf das Trägermaterial aufgesputtert. Als Element sind beispielsweise Titan, Silicium, Zirkonium, Aluminium und Zink zu nennen. Weitere Details sind der EP-B 0 564 830 zu entnehmen, deren diesbezüglicher Inhalt vollumfänglich in den Kontext der vorliegenden Anmeldung einbezogen wird.

In einigen Fällen kann auch eine, ebenfalls in der EP-B 0 564 830 beschriebene Hochvakuumbedampfung (beispielsweise Elektronenstrahl) verwendet werden.

Die strukturierten Träger können entweder vor oder nach der Aufbringung der Aktivmetalle bzw. Promotoren beispielsweise mittels einer Zahnradwelle verformt bzw. zusammengerollt werden, um ein monolithisches Katalysatorelement zu erhalten.

Die erfindungsgemäß verwendeten Katalysatoren können technisch hergestellt werden durch Auftragen mindestens eines Metalls der VIII. Nebengruppe des Periodensystems und gegebenenfalls mindestens eines Promotors auf einen der vorstehend beschriebenen Träger.

Die Aufbringung der Aktivmetalle und gegebenenfalls Promotoren auf den vorstehend beschriebenen Trägern kann erfolgen, indem man die Aktivmetalle im Vakuum verdampft und kontinuierlich auf den Träger kondensiert. Eine andere Möglichkeit besteht darin, die Aktivmetalle durch Tränken mit Lösungen, welche die Aktivmetalle und gegebenenfalls Promotoren enthalten, auf den Trägem aufzubringen. Eine weitere Möglichkeit besteht darin, die Aktivmetalle und gegebenenfalls Promotoren durch chemische Methoden, wie der Chemical Vapour Deposition (CVD) auf den Trägem aufzubringen.

Die so hergestellten Katalysatoren können direkt eingesetzt werden oder vor ihrem Einsatz getempert und/oder calciniert werden, und können sowohl vorreduziert als auch im nichtreduzierten Zustand eingesetzt werden.

Wahlweise wird der Träger vor der Aufbringung der Aktivmetalle und gegebenenfalls Promotoren vorbehandelt. Eine Vorbehandlung ist beispielsweise vorteilhaft, wenn die Haftung der Aktivkomponenten auf dem Träger verbessert werden soll. Beispiele für eine Vorbehandlung sind die Beschichtung des Trägers mit Haftvermittlern oder eine Aufrauhung mit mechanischen (etwa Schleifen, Sandstrahlen) oder thermischen Verfahren wie Erhitzen, in der Regel an Luft, Plasmaätzen oder Glimmen.

In einer bevorzugten Ausführungsform betrifft die vorliegende Erfindung ein derartiges Verfahren, wobei der Katalysator als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.-%, bezogen auf das Gesamtgewicht des Katalysators, beträgt (Katalysator 1). Weiter bevorzugt beträgt der mittlere Porendurchmesser des Trägers in diesem Katalysator mindestens 0,1 µm und die BET-Oberfläche höchstens 15 m²/g (Katalysator 1a).

Ferner betrifft sie ein derartiges Verfahren, wobei der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert (Katalysator 2).

Als Träger können prinzipiell alle Träger eingesetzt werden, die Makroporen aufweisen, d.h. Träger, die ausschließlich Makroporen aufweisen sowie solche, die neben Makroporen auch Meso- und/oder Mikroporen enthalten.
Die Begriffe *"Makroporen"* und *"Mesoporen"* werden im Rahmen der vorliegenden Erfindung so verwendet, wie sie in *Pure Appl. Chem*., *45*, *S. 79 (1976)* definiert sind, nämlich als Poren, deren Durchmesser oberhalb von 50 nm (Makroporen) oder deren Durchmesser zwischen 2 nm und 50 nm liegt (Mesoporen). *"Mikroporen* " sind ebenfalls entsprechend der obigen Literatur definiert und bezeichnen Poren mit einem Durchmesser von < 2 nm.

Der Gehalt des Aktivmetalls beträgt im allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei den im folgenden beschriebenen, vorzugsweise eingesetzten Katalysatoren 1 bis 2 vorzugsweise verwendeten Gehalte nochmals bei der Diskussion dieser Katalysatoren einzeln angegeben sind.

Im folgenden sollen nunmehr die vorzugsweise verwendeten Katalysatoren 1 und 2 detailliert beschrieben werden. Dabei erfolgt die Beschreibung beispielhaft unter Bezugnahme auf die Verwendung von Ruthenium als Aktivmetall. Die untenstehenden Angaben sind auch auf die anderen verwendbaren Aktivmetalle, wie hierin definiert, übertragbar.

### Katalysator 1

Die erfindungsgemäß verwendeten Katalysatoren 1 können technisch hergestellt werden durch Auftragen mindestens eines Metalls der VIII. Nebengruppe des Periodensystems und gegebenenfalls mindestens eines Metalls der I. oder VII. Nebengruppe des Periodensystems auf einem geeigneten Träger.

Die Auftragung kann durch Tränken des Trägers in wäßrigen Metallsalzlösungen, wie z.B. wäßrigen Rutheniumsalzlösungen, durch Aufsprühen entsprechender Metallsalzlösungen auf den Träger oder durch andere geeignete Verfahren erreicht werden. Als Metallsalze der L, VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend, vorzugsweise bei Temperaturen von 100 bis 150 °C, getrocknet und wahlweise bei Temperaturen von 200 bis 600 °C, vorzugsweise von 350 bis 450 °C calciniert. Bei getrennter Auftränkung wird der Katalysator nach jedem Tränkschritt getrocknet und wahlweise calciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen von ungefähr 30 bis ungefähr 600 °C, vorzugsweise von ungefähr 150 bis ungefähr 450 °C aktiviert. Vorzugsweise besteht der Gasstrom aus 50 bis 100 Vol.-% H₂ und 0 bis 50 Vol.-% N₂.

Die Metallsalzlösung oder -lösungen werden in einer solchen Menge auf den oder die Träger aufgebracht, daß der Gesamtgehalt an Aktivmetall, jeweils bezogen auf das Gesamtgewicht des Katalysators, ungefähr 0,01 bis ungefähr 30 Gew.-%, vorzugsweise ungefähr 0,01 bis ungefähr 5 Gew.-%, weiter bevorzugt ungefähr 0,01 bis ungefähr 1 Gew.-%, und insbesondere ungefähr 0,05 bis ungefähr 1 Gew.-% beträgt.

Die Metalloberfläche auf dem Katalysator 1 beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. Lemaitre et al. in *"Characterization of Heterogeneous Catalysts"*, Hrsg. Francis Delanney, Marcel Dekker, New York 1984, S. 310 - 324, beschriebenen Chemisorptionsverfahren bestimmt.

Im erfindungsgemäß verwendeten Katalysator 1 beträgt das Verhältnis der Oberflächen des/der Aktivmetalls/-metalle und des Katalysatorträgers vorzugsweise weniger als ungefähr 0,05, wobei der untere Grenzwert bei ungefähr 0,0005 liegt.

Die zur Herstellung der erfindungsgemäß verwendeten Katalysatoren verwendbaren Trägermaterialien sind solche, die makroporös sind und einen mittleren Porendurchmesser von mindestens ungefähr 50 nm, vorzugsweise mindestens ungefähr 100 nm, insbesondere mindestens ungefähr 500 nm aufweisen und deren Oberfläche nach BET bei höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt. Der mittlere Porendurchmesser des Trägers beträgt vorzugsweise ungefähr 100 nm bis ungefähr 200 µm, weiter bevorzugt ungefähr 500 nm bis ungefähr 50 µm. Die Oberfläche nach BET des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET-Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Markoporen aufweisende Aktivkohle, Siliciumcarbid, Aluminiumoxid, Siliciumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 196 24 484.6 zu entnehmen, deren diesbezüglicher Inhalt durch Bezugnahme vollständig in die vorliegende Anmeldung einbezogen wird.

Die zur Herstellung der erfindungsgemäß verwendeten Katalysatoren 1a, der eine bevorzugte Ausführungsform des Katalysators 1 darstellt, verwendbaren Trägermaterialien sind solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g aufweisen. Bevorzugt liegt der mittlere Porendurchmesser des dort verwendeten Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g. Auch dieser Katalysator weist bzgl. der Porendurchmesserverteilung die bereits oben beschriebene Bimodalität mit den analogen Verteilungen und das entsprechende bevorzugte Porenvolumen auf. Weitere Details bezüglich Katalysator 1a sind der DE-A 196 04 791.9 zu entnehmen, deren diesbezüglicher Inhalt durch Bezugnahme vollständig in die vorliegende Anmeldung einbezogen wird.

### Katalysator 2

Die erfindungsgemäß verwendeten Katalysatoren 2 enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die erfindungsgemäß verwendeten Katalysatoren 2 können technisch hergestellt werden durch Auftragen mindestens eines Aktivmetalls der VIII. Nebengruppe des Periodensystems, vorzugsweise Ruthenium und gegebenenfalls mindestens eines Metalls der I oder VII. Nebengruppe des Periodensystems auf einem geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen von 100 bis 150 °C bevorzugt sind. Wahlweise können diese Träger bei Temperaturen von 200 bis 600 °C, vorzugsweise von 350 bis 450 °C calciniert werden. Anschließend werden die beschichteten Träger durch Behandlung in einem Gasstrom, der freien Wasserstoff enthält, bei Temperaturen von 30 bis 600 °C, vorzugsweise von 100 bis 450 °C und insbesondere von 100 bis 300 °C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Aufragen nacheinander, so kann der Träger nach jedem Auftragen bzw. Tränken bei Temperaturen von 100 bis 150 °C getrocknet werden und wahlweise bei Temperaturen von 200 bis 600 °C calciniert werden. Dabei kann die Reihenfolge, in der die Metallsalzlösung aufgetragen oder aufgetränkt wird, beliebig gewählt werden.

Die Metallsalzlösung wird in einer solchen Menge auf den/die Träger aufgebracht, daß der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. Lemaitre et al., *"Characterization of Heterogeneous Catalysts",* Hrsg. Francis Delanney, Marcel Dekker, New York (1984), S. 310 - 324, beschrieben ist.

Im erfindungsgemäß verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß verwendbaren Träger eine Porenverteilung auf, dergemäß ungefähr 5 bis ungefähr 50%, vorzugsweise ungefähr 10 bis ungefähr 45%, weiter bevorzugt ungefähr 10 bis ungefähr 30 und insbesondere ungefähr 15 bis ungefähr 25% des Porenvolumens von Makroporen mit Porendurchmessern im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Anteile der Porenvolumina zu 100% addiert.

Das Gesamtporenvolumen der erfindungsgemäß verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysator 2 sind der DE-A 196 24 485.4 zu entnehmen, deren diesbezüglicher Inhalt durch Bezugnahme vollständig in die vorliegende Anmeldung einbezogen wird.

Weitere Details bezüglich der in dem erfindungsgemäßen Verfahren verwendeten Katalysatoren, deren Aufbau und Herstellung sind in der DE 199 17 051.7 zu entnehmen, deren diesbezüglicher Inhalt durch Bezugnahme vollständig in die vorliegende Anmeldung einbezogen wird.

Die bei der Reaktion entstehenden leichtsiedenden Nebenprodukte, werden während der Reaktivdestillation - gegebenenfalls als Azeotrop mit den Edukten - über Kopf abdestilliert und aus dem Reaktionssystem ausgeschleust. Analog hierzu werden gegebenenfalls entstehende hochsiedende Nebenprodukte über Sumpf abgetrennt.

Die bei der stark exothermen Reaktion freiwerdende Energie wird zur Destillation genutzt.

Bei dem erfindunsgemäßen Verfahren können neben Benzol auch dessen substituierte Derivate wie beispielsweise Toluol oder Xylol zu den entsprechenden gesättigten Kohlenwasserstoffen umgesetzt werden.

Innerhalb des erfindungsgemäßen Verfahrens können prinzipiell alle ein- oder mehrkemigen Aromaten, die entweder unsubstituiert sind oder mit mindestens einer Alkylgruppe, Aminogruppe oder Hydroxylgruppe oder einer Kombination aus zwei oder mehr davon stubstituiert sind, einzeln oder als Gemische aus zwei oder mehr davon, vorzugsweise einzeln eingesetzt werden. Die Länge der Alkylgruppe unterliegt dabei auch keinerlei besonderen Beschränkungen, im allgemeinen handelt es sich jedoch um C₁- bis C₃₀-, vorzugsweise C₁- bis C₁₈-, insbesondere C₁- bis C₄-Alkylgruppen.

Ferner können erfindungsgemäß aromatische Verbindungen, in denen mindestens eine Hydroxylgruppe und vorzugsweise ferner mindestens ein gegebenenfalls substituierter C₁₋ ₁₀-Alkylrest und/oder Alkoxyrest an einen aromatischen Kern gebunden ist, zu den entsprechenden cycloaliphatischen Verbindungen hydriert werden, wobei auch Gemische zweier oder mehr dieser Verbindungen eingesetzt werden können.

Dabei können die aromatischen Verbindungen einkernige oder mehrkernige aromatische Verbindungen sein. Die aromatischen Verbindungen können mindestens eine Hydroxylgruppe enthalten, die an einen aromatischen Kern gebunden ist, die einfachste Verbindung dieser Gruppe ist Phenol. Vorzugsweise weisen die aromatischen Verbindungen eine Hydroxylgruppe pro aromatischem Kern auf. Die aromatischen Verbindungen können an dem aromatischen Kern oder den aromatischen Kernen substituiert sein durch einen oder mehrere Alkyl- und/oder Alkoxyreste, vorzugsweise C₁₋₁₀-Alkyl- und/oder Alkoxyreste, besonders bevorzugt C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Vorzugsweise weisen die erfindungsgemäß hydrierbaren Verbindungen mindestens einen, vorzugsweise einen bis vier, insbesondere einen C₁₋₁₀-Alkylrest auf, der sich vorzugsweise am gleichen aromatischen Kern befindet wie die mindestens eine Hydroxylgruppe. Bevorzugte Verbindungen sind (Mono)alkylphenole, wobei der Alkylrest in o-, m- oder p-Position zur Hydroxylgruppe stehen kann. Insbesondere bevorzugt sind trans-Alkylphenole, auch als 4-Alkylphenole bezeichnet, wobei der Alkylrest vorzugsweise 1 bis 10 Kohlenstoffatome aufweist und insbesondere ein tert.-Butylrest ist. Bevorzugt ist 4-tert.-Butylphenol. Erfindungsgemäß verwendbare mehrkernige aromatische Verbindungen sind beispielsweise β-Naphthol und α-Naphthol.

Die aromatischen Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, können auch mehrere aromatische Kerne aufweisen, die über einen Alkylenrest, vorzugsweise eine Methylengruppe verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt sind Methyl-, Ethyl-, Propyl-, Isopropyl-, Butyl- oder tert.-Butylreste.

Dabei kann jeder der aromatischen Kerne mindestens eine Hydroxylgruppe gebunden enthalten. Beispiele solcher Verbindungen sind Bisphenole, die in 4-Position über einen Alkylenrest, vorzugsweise einen Methylenrest, verknüpft sind.

Insbesondere bevorzugt hydriert wird im Rahmen des erfindungsgemäßen Verfahrens ein mit einem C₁₋₁₀-Alkylrest, vorzugsweise C₁₋₆-Alkylrest, substituiertes Phenol, wobei der Alkylrest gegebenenfalls mit einem aromatischen Rest substituiert ist, oder Gemische zweier oder mehrerer dieser Verbindungen.

In einer weiteren bevorzugten Ausführungsform dieses Verfahrens wird p-tert.-Butylphenol, Bis(p-hydroxyphenyl)dimethylmethan oder ein Gemisch davon hydriert.

Mit dem erfindungsgemäßen Verfahren können ferner aromatische Verbindungen, in denen mindestens eine Aminogruppe an einen aromatischen Kern gebunden ist, zu den entsprechenden cycloaliphatischen Verbindungen hydriert werden, wobei auch Gemische zweier oder mehr dieser Verbindungen eingesetzt werden können. Dabei können die aromatischen Verbindungen einkernige oder mehrkernige aromatische Verbindungen sein. Die aromatischen Verbindungen enthalten mindestens eine Aminogruppe, die an einen aromatischen Kern gebunden ist. Vorzugsweise sind die aromatischen Verbindungen aromatische Amine oder Diamine. Die aromatischen Verbindungen können an dem aromatischen Kern oder den aromatischen Kernen oder an der Aminogruppe substituiert sein durch einen oder mehrere Alkyl- und/oder Alkoxyreste, vorzugsweise C₁₋₂₀-Alkylreste, insbesondere Methyl-, Ethyl-, Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Die aromatische Verbindung, in der mindestens eine Aminogruppe an einen aromatischen Kern gebunden ist, kann auch mehrere aromatische Kerne aufweisen, die über eine Alkylengruppe, vorzugsweise eine Methylengruppe, verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt Methyl-, Ethyl-, Propyl-, Isopropyl-, Butyl-, sec.-Butyl- oder tert.-Butylreste, sind.

Die an den aromatischen Kern gebundene Aminogruppe kann ebenfalls durch einen oder zwei der vorstehend beschriebenen Alkylreste substituiert sein.

Besonders bevorzugte Verbindungen sind Anilin, Naphthylamin, Diaminobenzole, Diaminotoluole und Bis-p-aminophenylmethan oder Gemische davon.

Im einzelnen werden im Rahmen des vorliegenden Verfahrens insbesondere die folgenden Aromaten hydriert: Benzol, Toluol, Xylole, Cumol, Diphenylmethan, Tri-, Tetra-, Pentaund Hexabenzole, Triphenylmethan, alkylsubstituierte Naphthaline, Naphthalin, alkylsubstituierte Anthracene, Anthracen, alkylsubstituierte Tetraline und Tetralin, sowie Anilin. Vorzugsweise wird im Rahmen der vorliegenden Verfahren Benzol zu Cyclohexan hydriert.

Obwohl die Hydrierung der Aromaten derart durchgeführt werden kann, daß das Wasserstoff enthaltende Gas und der/die flüssigen Aromaten im Gleichstrom in einer Kolonne geführt werden, wird die erfindungsgemäße Hydrierung vorzugsweise derart durchgeführt, daß das Wasserstoff enthaltende Gas im Gegenstrom zu dem/den flüssigen Aromaten in einer Kolonne geführt wird, die mit einem der vorstehend beschriebenen Katalysatoren bestückt ist. Hierbei kann die flüssige Phase von oben nach unten durch die Kolonne geführt werden und die gasförmige Phase von unten nach oben. Vorzugsweise wird die Hydrierung zwei- oder mehrstufig durchgeführt. Der in dieser Anmeldung beschriebene Katalysator wird hierbei in mindestens einer Stufe eingesetzt.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die erfindungsgemäße Hydrierung kann in Ab- oder Anwesenheit eines Lösungs- oder Verdünnungsmittels durchgeführt werden, d.h. es ist nicht erforderlich, die Hydrierung in Lösung durchzuführen.

Als Lösungs- oder Verdünnungsmittel kann jedes geeignete Lösungs- oder Verdünnungsmittel eingesetzt werden. Die Auswahl ist dabei nicht kritisch, solange das eingesetzte Lösungs- oder Verdünnungsmittel in der Lage ist, mit dem zu hydrierenden Aromaten eine homogene Lösung zu bilden

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung des zur Hydrierung vorgesehenen Aromaten führen.

Bei Verwendung eines Lösungsmittels wird im Rahmen des erfindungsgemäßen Verfahrens das bei der Hydrierung gebildete Produkt, also der oder die jeweilige(n) Cycloaliphat(en), als bevorzugte(s) Lösungsmittel eingesetzt, gegebenenfalls neben anderen Lösungs- oder Verdünnungsmitteln. In diesem Fall kann ein Teil des im Verfahren gebildeten Produkts dem noch zu hydrierenden Aromaten beigemischt werden.

Das vorliegende, erfindungsgemäße Verfahren weist zahlreiche Vorteile gegenüber den Verfahren des Standes der Technik auf. Die Reaktivdestillation kombiniert die Durchführung chemischer Reaktionen und die destillative Trennung der Edukte und Produkte in einer Vorrichtung. Dies bietet verfahrenstechnische Vorteile hinsichtlich der Reaktionsführung und senkt den Energieverbrauch. Zudem ergeben sich gegenüber der Durchführung von Reaktion und Destillation in getrennten Apparaturen Einsparungen bei den Investitionskosten.

Darüber hinaus können durch das erfindungsgemäße Verfahren die Aromaten bei deutlich niedrigeren Drücken und Temperaturen als im Stand der Technik beschrieben, selektiv und mit hoher Raum-Zeit-Ausbeute zu den entsprechenden Cycloaliphaten hydriert werden. Selbst bei niedrigeren Drücken und Temperaturen weisen die Katalysatoren eine hohe Aktivität auf. Die Cycloaliphaten werden in hochreiner Form erhalten. Es können selbst bei geringen Drücken Cycloaliplrate mit hoher Raum-Zeit-Ausbeute erhalten werden. Die Hydrierung kann des weiteren ohne den Zusatz von Hilfschemikalien mit ausgezeichneter Selektivität durchgeführt werden.

Figur 1 zeigt ein vereinfachtes Schemas einer Destillationsvorrichtung zur Durchführung des erfindungsgemäßen Verfahrens, in der der Cycloaliphat im Sumpf der Kolonne anfällt.

Figur 2 zeigt ein derartiges vereinfachtes Schema einer Destillationsvorrichtung zur Durchführung des erfindungsgemäßen Verfahrens, bei der der Cycloaliphat über einen Seitenabzug entnommen wird.

Die Figuren sollen nunmehr beispielshaft für die Herstellung von Cyclohexan, ausgehend vom Benzol näher erläutert werden.

Bei dem erfindungsgemäßen Verfahren wird gemäß Fig. 1 die Reaktion in einer Reaktionskolonne 4 an einem heterogenen Katalysator 5, wie er oben beschrieben wurde, mittels Reaktivdestillation durchgeführt. Die Zufuhrstelle 1 für Benzol mündet in den oberen Teil 3 der Reaktionskolonne 4 und die Zufuhrstelle 2 für Wasserstoff mündet in den unteren Teil der Reaktionskolonne 4. Auf diese Weise wird ein Gegenstrom der Reaktanden innerhalb der Reaktionskolonne 4 erzeugt. Benzol reagiert an dem heterogenen Katalysator 5 bei gleichzeitiger Destillation zu Cyclohexan. Cyclohexan ist der Schwersieder des Stoffsystems, destilliert in den Kolonnensumpf 6 und wird durch die Leitung 8 abgeführt.

Da Benzol und Cyclohexan ein Leichtsiederazeotrop bilden, wird das Konzentrationsprofil bei dem erfindungsgemäßen Verfahren so eingestellt, daß sich im Kolonnensumpf 6 kein Benzol befindet und sich ein Bereich hoher Konzentration an Benzol bzw. Benzol/Cyclohexan an dem heterogenen Katalysator 5.

Die bei der Reaktion entstehenden Nebenprodukte stellen Leichtsieder dar und werden, gegebenenfalls als Azeotrop mit Benzol oder Cyclohexan, am Kopfkondensator 9 auskondensiert. Der überwiegende Teil des Benzol enthaltenden Kopfstroms wird als Rücklauf 10 auf die Reaktionskolonne 4 gegeben und ein geringer Teil 7 des Kopfstroms, der die Nebenprodukte enthält, wird ausgeschleust. Darüber hinaus können im Benzol vorhandene leicht siedende Verunreinigungen ebenfalls auf einfache Weise vor der Reaktionszone mit dem heterogenen Katalysator 5 abgetrennt und über den Teil 7 des Kopfstroms ausgeschleust werden.

Zusammen mit den leichter siedenden Komponenten verläßt nicht umgesetzter, am Kopf der Reaktionskolonne 4 anfallender Wasserstoff 11 die Reaktionskolonne 4, der gegebenenfalls nach Ausschleusung eines Partialstroms 12 mittels eines Kompressors 13 in den Sumpf 6 der Reaktionskolonne 4 zurückgeführt wird.

Bei der Durchführung des erfindungsgemäßen Verfahrens mittels einer Vorrichtung, wie sie in Fig. 2 gezeigt ist, wird der Cycloaliphat als Wertprodukt, hier Cyclohexan, über einen Seitenabzug 14, der sich im unteren Teil der Kolonne 3b befindet, entnommen. Über die Leitung 8 im Kolonnensumpf 6 werden bei dieser Ausführungsform die Hochsieder erhalten. Im Unterschied zu Fig. 1 ist gemäß Fig. 2 der obere Teil der Kolonne mit 3a bezeichnet; ansonsten entsprechen die Bezeichnungen der Fig. 2 denen der Fig. 1.

Die Erfindung soll nun anhand der folgenden Beispiele näher erläutert werden:

### Beispiel

### Katalysator A:

Bei diesem Katalysator handelte es sich um einen im Handel erhältlichen Katalysator mit 0,5 % Ruthenium auf Al₂O₃-Kugeln mit Meso- und Makroporen entsprechend dem Katalysator 2 der vorliegenden Erfindung.

### Katalysator B:

Die katalytische Packung dieses Katalysators wurde aus Metallgewebe hergestellt, das vorher mit Ruthenium beschichtet worden war. Das Herstellungsverfahren ist in der Druckschrift EP-A 0 564 830 beschrieben, deren diesbezüglicher Inhalt durch die Inbezugnahme in die vorliegende Anmeldung übernommen wird.

### Durchführung des Verfahrens

Die Versuchsvorrichtung bestand aus einem beheizbaren, mit Rührer ausgestatteten 2-Liter-Reaktionskolben aus Edelstahl, auf den eine aus 2 Kolonnenschüssen bestehende Destillationskolonne (Länge: 1 m; Durchmesser: 50 mm) aufgesetzt war. Die Destillationskolonne war im unteren Bereich (0,5 m) einmal mit dem vorangehend beschriebenen Katalysator A und in einem anderen Versuch mit dem Katalysator B befüllt, während der obere Bereich der Destillationskolonne mit einer Destillationspackung des Typs Montz A3-750 beschickt war. Das Benzol wurde mittels einer Pumpe auf die oberste Stufe der Destillationskolonne dosiert. Der Wasserstoff wurde in die Destillationsblase dosiert. Auf diese Weise wurde ein Gegenstrom der Reaktanden über den Katalysator realisiert.

Der Wasserstoff und entstehende Nebenprodukte wurde über die Reaktionskolonne abgetrennt und in einen Teilkondensator kondensiert. Das Kondensat lief über einen Rücklaufteiler in einen Vorlagebehälter. Der verbleibende Abgasstrom wurde durch eine Kühlfalle und anschließend zur Volumenmessung durch eine Gasuhr geleitet.

Die Vorrichtung war mit einer Druckregelung ausgestattet und auf einen Systemdruck von 20 bar ausgelegt.

Alle ein- und austretenden Stoffströme wurden während der Versuchsdauer kontinuierlich erfaßt und registriert, so daß eine zeitabhängige Massenbilanzierung möglich war.

Alternativ dazu wurde mit derselben Verfahrensvorrichtung ein Vergleichsversuch in Rieselfahrweise durchgeführt.

Die Versuchsbedingungen und Versuchsergebnisse sind in Tabelle 1 zusammengestellt.

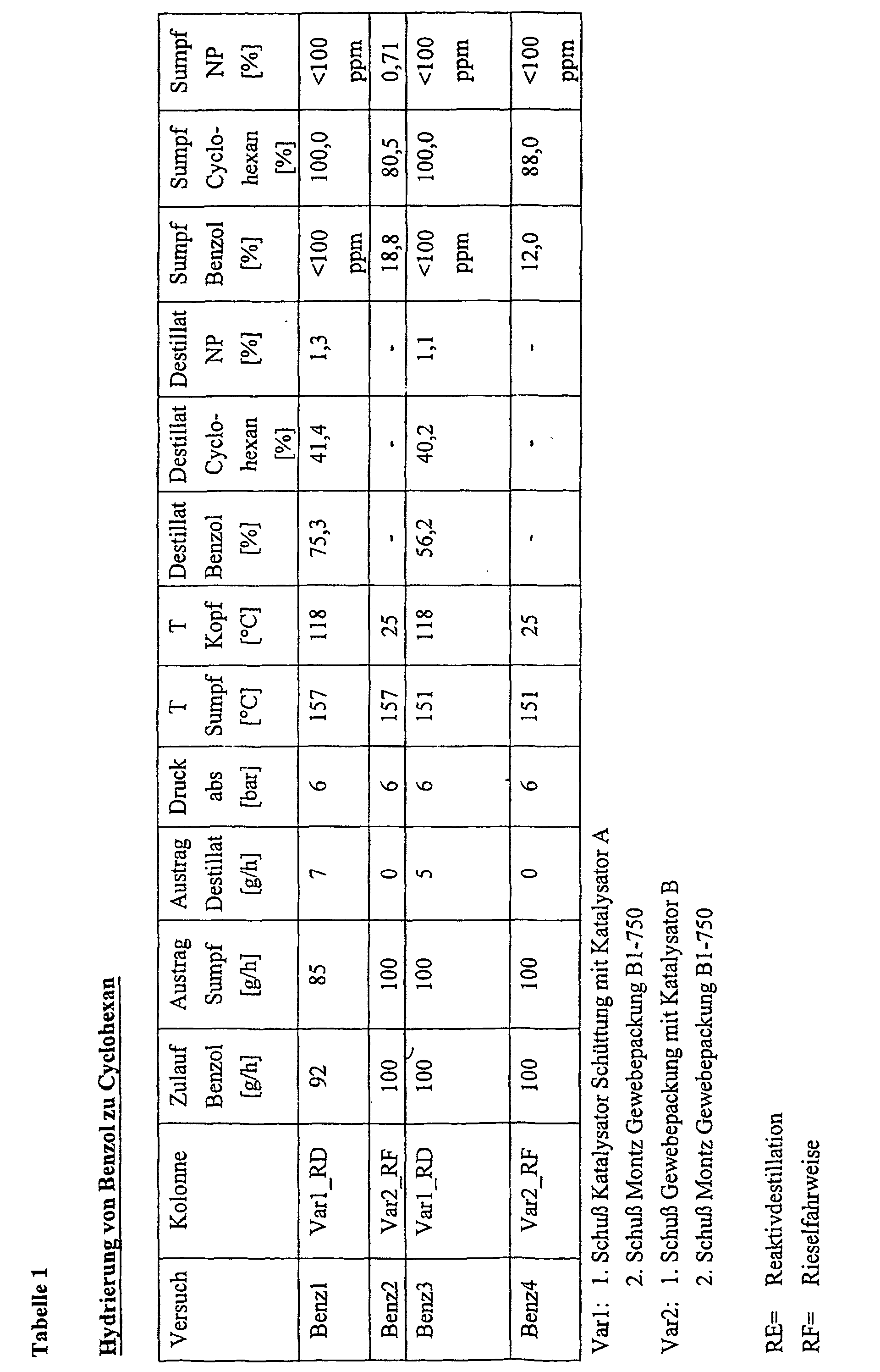

## Patentansprüche

1. Verfahren zur Hydrierung von mit mindestens einer Alkylgruppe, Aminogruppe oder Hydroxylgruppe oder einer Kombination aus zwei oder mehr davon substituierten oder unsubstituierten ein- oder mehrkernigen Aromaten zu den entsprechenden Cycloaliphaten mit gasförmigem Wasserstoff in Gegenwart mindestens eines Katalysators in einer Reaktionskolonne unter Führung der Reaktionspartner über den(die) in der Reaktionskolonne fixierten Katalysatoren), wobei die Hydrierung derart durchgeführt wird, daß die Cycloaliphaten aus einem Seitenabzug oder dem Sumpf der Kolonne oder dem Seitenabzug und dem Sumpf der Kolonne entnommen werden.

2. Verfahren nach Anspruch 1, **dadurch gekennzeichnet, daß** die Reaktionspartner im Gegenstrom über den(die) in der Reaktionskolonne fixierten Katalysator(en) geführt werden.

3. Verfahren nach Anspruch 1 oder 2, worin die Hydrierung bei einem Druck < 20 bar und bei einer Temperatur < 200 °C durchgeführt wird.

4. Verfahren nach einem der Ansprüche 1 bis 3, worin die Hydrierung bei einem Druck < 13 bar und bei einer Temperatur < 150 °C durchgeführt wird.

5. Verfahren nach einem der Ansprüche 1 bis 4, worin als Katalysator ein heterogener Katalysator verwendet wird.

6. Verfahren nach Anspruch 5, worin als Katalysator ein Ruthenium-Katalysator verwendet wird.

7. Verfahren nach Anspruch 5 oder Anspruch 6, worin als Katalysator ein Ruthenium-Katalysator in Form einer Schüttung in der Reaktionskolonne und/oder in Form einer Destillationspackung in der Kolonne, vorzugsweise in Form einer aus anorganischen oder organischen Fäden bestehenden, mit Ruthenium beschichteten Destillationspackung verwendet wird.

8. Verfahren nach einem der Ansprüche 1 bis 7, worin die Hydrierung bei einem Druck im Bereich von 1 bis 20 bar, vorzugsweise 5 bis 13 bar, und/oder bei einer Temperatur im Bereich von 50 bis 200 °C, vorzugsweise 80 bis 150 °C, durchgeführt wird.

9. Verfahren nach einem der Ansprüche 1 bis 8, worin bei der Hydrierung der Wasserstoff-Partialdruck im Bereich von 0,1 bis 20 bar, vorzugsweise 5 bis 13 bar, liegt.

10. Verfahren nach einem der Ansprüche 1 bis 9, worin nicht erwünschte Nebenprodukte, während der Reaktivdestillation über Kopf abgetrennt werden.

11. Verfahren nach einem der Ansprüche 1 bis 10, wobei Cyclohexan aus Benzol, Methylcyclohexan aus Toluol, Dimethylcyclohexan aus Xylol, oder Cyclohexylamin aus Anilin hergestellt wird.

## Claims

1. A process for hydrogenating unsubstituted monocyclic or polycyclic aromatics or monocyclic or polycyclic aromatics substituted by at least one alkyl group, amino group or hydroxyl group or a combination of two or more thereof to form the corresponding cycloaliphatics by means of gaseous hydrogen in the presence of at least one catalyst in a reaction column in which the reactants are passed over the catalyst(s) fixed in the reaction column, wherein the cycloaliphatics are taken off at a side offtake or from the bottom of the column or at the side offtake and from the bottom of the column.

2. A process as claimed in claim 1, wherein the reactants are passed in countercurrent over the catalyst(s) fixed in the reaction column.

3. A process as claimed in claim 1 or 2, wherein the hydrogenation is carried out at a pressure of < 20 bar and a temperature of < 200°C.

4. A process as claimed in any of claims 1 to 3, wherein the hydrogenation is carried out at a pressure of < 13 bar and a temperature of < 150°C.

5. A process as claimed in any of claims 1 to 4, wherein a heterogeneous catalyst is used.

6. A process as claimed in claim 5, wherein a ruthenium catalyst is used.

7. A process as claimed in claim 5 or claim 6, wherein a ruthenium catalyst in the form of a bed in the reaction column and/or in the form of distillation packing in the column, preferably in the form of ruthenium-coated distillation packing comprising inorganic or organic threads, is used.

8. A process as claimed in any of claims 1 to 7, wherein the hydrogenation is carried out at a pressure in the range from 1 to 20 bar, preferably from 5 to 13 bar, and/or at a temperature in the range from 50 to 200°C, preferably from 80 to 150°C.

9. A process as claimed in any of claims 1 to 8, wherein the hydrogen partial pressure during the hydrogenation is in the range from 0.1 to 20 bar, preferably from 5 to 13 bar.

10. A process as claimed in any of claims 1 to 9, wherein undesired by-products are separated off via the top during the reactive distillation.

11. A process as claimed in any of claims 1 to 10, wherein cyclohexane is prepared from benzene, methylcyclohexane is prepared from toluene, dimethylcyclohexane is prepared from xylene, or cyclohexylamine is prepared from aniline.

## Revendications

1. Procédé pour l'hydrogénation de composés aromatiques mononucléaires ou polynucléaires non substitués ou substitués avec au moins un groupe alkyle, un groupe amino ou un groupe hydroxyle ou avec une combinaison de deux desdits groupes ou plus pour obtenir les composés cycloaliphatiques correspondants, avec de l'hydrogène gazeux en présence d'au moins un catalyseur dans une colonne de réaction en guidant les partenaires réactionnels par-dessus le ou les catalyseur(s) fixé(s) dans la colonne de réaction, l'hydrogénation étant mise en oeuvre de telle sorte que l'on récupère les composés cycloaliphatiques à partir d'un dégagement latéral ou de bas de colonne ou bien à partir du dégagement latéral et de bas de colonne.

2. Procédé selon la revendication 1, **caractérisé en ce que** les partenaires réactionnels sont guidés à contre-courant par-dessus le ou les catalyseur(s) fixé(s) dans la colonne de réaction.

3. Procédé selon la revendication 1 ou 2, dans lequel on effectue l'hydrogénation sous une pression < 20 bars et à une température < 200 °C.

4. Procédé selon l'une quelconque des revendications 1 à 3, dans lequel on effectue l'hydrogénation sous une pression < 13 bars et à une température < 150 °C.

5. Procédé selon l'une quelconque des revendications 1 à 4, dans lequel on utilise, à titre de catalyseur, un catalyseur hétérogène.

6. Procédé selon la revendication 5, dans lequel on utilise, à titre de catalyseur, un catalyseur à base de ruthénium.

7. Procédé selon la revendication 5 ou la revendication 6, dans lequel on utilise, à titre de catalyseur, un catalyseur à base de ruthénium, sous la forme d'une matière en vrac dans la colonne de réaction et/ou sous la forme d'un garnissage de distillation dans la colonne, de préférence sous la forme d'un garnissage de distillation constitué par des fils inorganiques ou organiques, enduit de ruthénium.

8. Procédé selon l'une quelconque des revendications 1 à 7, dans lequel on effectue l'hydrogénation sous une pression dans la plage de 1 à 20 bars, de préférence de 5 à 13 bars et/ou à une température dans la plage de 50 à 200 °C, de préférence de 80 à 150 °C.

9. Procédé selon l'une quelconque des revendications 1 à 8, dans lequel, au cours de l'hydrogénation, la pression partielle d'hydrogène se situe dans la plage de 0,1 à 20 bars, de préférence de 5 à 13 bars.

10. Procédé selon l'une quelconque des revendications 1 à 9, dans lequel des sous-produits non désirés sont séparés via la tête de la colonne au cours de la distillation par réaction.

11. Procédé selon l'une quelconque des revendications 1 à 10, dans lequel on prépare du cyclohexane à partir du benzène, du méthylcyclohexane à partir du toluène, du diméthylcyclohexane à partir du xylène, ou de la cyclohexylamine à partir d'aniline.
